# EUROPEAN PATENT APPLICATION

(11) **EP 1 690 824 A1**
(43) Date of publication of application: **16.08.2006**
(21) Application number: 06002646.5
(22) Date of filing: 09.02.2006
(51) Int. Cl.: B66F 17/00

(54) **Mobile work platform**

(30) Priority: 11.02.2005 GB 0502864
(71) Applicant: Niftylift Limited, Stonebridge Milton Keynes MK13 0ER (GB)
(72) Inventor: Bowden, Frank Roger, Totternhoe LU6 2BY (GB)
(74) Representative: Raynor, Simon Mark

(57) **Abstract**

A mobile work platform that has a load detection system is disclosed. The platform comprises a cage (2) that is designed to receive one or more human operators, the cage (2) being carried on a boom (4) that is operable to vary the height of the cage (2). The boom includes a link component (16) that is movable by the weight of the cage and its contents against the action of resilient biasing means, which may include a helical compression spring (44). Detection means (48) is provided operative to generate an output signal in the event that displacement of the link component exceeds a predetermined threshold. For example, the detection means (48) may include a microswitch which preferably has multiple operative positions to enable operation of the platform to be altered in response to different degrees of excessive loading. The spring length may be adjusted by a bolt (46) in order to provide a predetermined compression force.

## Description

The present invention relates to a mobile work platform, and in particular to a mobile work platform having a load detection mechanism for an overload prevention system.

Mobile work platforms typically include a cage that is designed to receive one or more human operators. The cage is mounted on a hydraulically operated boom. The work platform is usually mounted on a wheeled chassis, which allows the platform to be moved easily to a desired location.

To ensure that the work platform remains safe, it must be provided with an overload protection system. The system is designed to provide a warning if the load in the cage exceeds a first set value and to prevent operation of the platform if the load exceeds a second, higher value.

Previous overload protection systems have relied on use of an electronic strain gauge to detect the load in the cage. However, strain gauges are expensive and easily damaged, and they can be over-ridden by a reckless operator (for example by jamming a screwdriver into the gap occupied by the strain gauge, to prevent it receiving the full load). They are also temperature-sensitive and suffer from drift, so requiring frequent recalibration of the system.

An aim of the invention is to provide a mobile work platform having a load detection mechanism that is simple, robust, relatively inexpensive and easy to maintain, yet which is accurate and reliable.

To this end, this invention provides a mobile work platform comprising a cage that is designed to receive one or more human operators, the cage being carried on a boom that is operable to vary the height of the cage, in which the boom includes a link component that is movable by the weight of the cage against the action of resilient biasing means, and detection means operative to generate an output signal in the event that displacement of the link component exceeds a predetermined threshold.

This arrangement is simple and can readily be made robust, while it is also difficult to circumvent.

The link component is conveniently a pivotable link that is part of a linkage that supports the cage. For example, it may be one of a plurality of parallel links that guide the cage for linear movement.

The actuation mechanism works in conjunction with the parallel links, and by virtue of the geometry of their construction, transfers a multiplying effect to the cage deflection, effectively increasing by a significant ratio, the accuracy and sensitivity of the detection system.

The resilient biasing means may be pre-stressed to prevent movement of the link component unless the weight of the cage exceeds a predetermined threshold. Movement of the link component only occurs when the weight of the cage exceeds the predetermined threshold. This movement can be sensed to detect that the cage is overloaded. By adjusting the pre-stress on the resilient biasing means, the threshold value can be easily adjusted. The mechanism is simple to adjust and is both robust and reliable. In the present context, "the weight of the cage" means the weight of the cage itself plus any contents of the cage.

The resilient biasing means may comprise a helical compression spring. The spring may be pre-loaded such that it is compressed even when the cage is unloaded. This prevents movement of the link component until the load of the cage exceeds a threshold. Loading may be achieved by respective plates abutting end surfaces of the spring, the plates being interconnected by a tie member that passes axially through the spring. The tie member therefore acts in tension. Preferably, the effective length of the tie member may be adjustable so that the pre-loading of the spring can be varied. This may, for example, be achieved by the tie member being threaded; for example, it may be a bolt.

Typically, the detection means has a number of different operating conditions, the active operating condition being determined by the load within the cage. As a simple example, it may have two operating conditions - on or off. This can be used to prevent operation of the platform when the load has exceeded a predetermined maximum. More advantageously, the detection means may have a number of operating conditions in excess of two. This enables a plurality of operating conditions to be defined, depending upon the degree of excess load present in the cage. For example, an alarm condition may be triggered for marginally excessive loading and operation of the cage may be prevented for greater excessive loading.

The detection means may suitably be an electrical device such as a microswitch. Alternatively, a non contact device such as a proximity sensor may be used.

Most advantageously, adjustment means may be provided whereby the relative positions of the detection means and the link component may be altered. This allows the detection means to operate correctly in the presence of dimensional variations resulting from manufacturing tolerances, or otherwise. For example, the adjustment means may comprise a screwed component of the link component. The screwed component may serve to operate the detection means, the position of the screwed component being adjustable with respect to other parts of the link component.

A stop may be provided to limit the motion of the link component towards the detection means to prevent the detection means being damaged in the event of severe overloading.

An embodiment of the invention will now be described, by way of example, with reference to the accompanying drawings, wherein:
Figure 1 is a partially sectional side elevation of a mobile work platform having a load detection mechanism;
Figure 2 is an exploded view showing certain parts of the mobile work platform; and
Figures 3a-3e are side views of a microswitch in various stages of operation.

The present invention relates to a mobile work platform having a load detection mechanism for detecting the load carried by the platform. The load detection mechanism forms part of an overload prevention system (not shown), which may typically also include a central controller that is connected to the load detection mechanism and controls operation of an overload warning alarm and a hydraulic drive system for the mobile work platform. Apart from the load detection mechanism, the overload prevention system may be conventional in structure and operation, and so will not be described in detail.

The mobile work platform includes a cage 2 that is designed to receive one or more human operators, which is mounted on the end of a hydraulically operated boom 4. The mobile work platform may be mounted on a wheeled chassis (not shown) which allows the platform to be moved easily to a desired location.

The cage 2 includes a floor panel 6 that is supported by a rigid cage support beam 8, and a set of railings 10 that surround the floor panel. The cage is connected to the end of the hydraulically operated boom 4 via a support frame 12 that has two sides, each comprising an outer frame element 14, an inner frame element 16 and a link arm 18. Both sides of the support frame are similar and in the remainder of this description references to one side of the support frame apply also to the other side.

The outer frame element 14 is pivotally attached via a first pivot pin 20 to one side of the boom 4 and via a second pivot pin 22 to a parallel link 24. The boom 4 and the parallel link 24 are conventional and ensure that during movement of the boom the support frame 12 and the cage 2 always remain at the same angle relative to the ground, so that the floor of the cage is always horizontal. In this embodiment, the link 24 is shown as a hydraulically operated levelling cylinder, which allows the length of the link to be matched to the boom length as the boom extends. If the boom has a fixed length, the link 24 may be a simple tie rod.

The cage 2 is connected to the outer frame elements 14 via the inner frame elements 16 and the connecting arms 18. Each inner frame element 16 is pivotally connected to the corresponding outer frame element 14 via a third pivot pin 26 and to the cage support beam 8 via a fourth pivot pin 28. The link arm 18 is connected to the outer frame element 14 and to the cage support beam 8 via fifth and sixth pivot pins 30, 32 respectively. The inner frame element 16 and the link arm 18 comprise parallel links, which together ensure that during movement of the boom, the cage always maintains the same angle relative to the support frame, so that the floor of the cage 2 is always horizontal. The parallel link arrangement provided by the inner frame element and the link arm also allows for vertical movement of the cage 2 relative to the support frame 12, through pivotal movement of those elements.

The inner frame element 16 (referred to in the claims as a "link component") is roughly triangular in shape and includes an actuator arm 34 that extends downwards from the region of the third and fourth pivot pins 26, 28. The two inner frame elements 16 are interconnected by means of a first connecting plate 36 located towards the ends of the arms, and a second connecting plate 38 located approximately half way along the length of the arms. Third and fourth connecting plates 40, 42 interconnect the two outer frame elements 14.

The actuator arm 34 rotates in an arc around the third pivot pin 26 in response to vertical movement of the cage 2 relative to the support frame 12. The arm thus rotates in the direction of arrow A when the cage moves downwards and in the opposite direction when the cage moves upwards. The length of the actuator arm 34 is greater than the distance between the two pivot pins 26,28. Any vertical movement of the cage 2 will thus produce a much larger movement at the free end of the actuator arm 34.

Vertical movement of the cage 2 relative to the support frame 12 is limited by a spring and bolt arrangement 44, 46, which extends between the second and fourth connecting plates 38, 42. The spring 44 is compressed between those connecting plates and the compression of the spring can be adjusted by tightening or loosening the bolt 46, which extends along the length of the spring within its helix. The bolt 46 restricts upwards movement of the cage 2 by preventing separation of the second and fourth connecting plates, while the compressed spring 44 opposes inwards movement of the second and fourth connecting plates caused by downwards movement of the cage 2. As the spring is stressed by tightening the bolt to create a compressive pre-loading, downwards movement of the cage is prevented unless the weight of the cage 2 and its contents is sufficient to overcome the compression of the spring 44.

The third connecting plate 40 supports a microswitch 48, which is located so that it can be actuated by an adjustable screw 50 that extends through the first connecting plate 36 at the lower end of the arm. A locknut 52 is carried on the adjustable screw 50 can be tightened to prevent movement of the adjustable screw 50. The microswitch 48 is therefore able to detect movement of the actuator arm 34, caused by downwards movement of the cage 2. The microswitch 48 is connected via electronic circuitry to an alarm and control system (not shown) for providing an audible and/or visual alarm in the event that the load carried by the cage approaches a predetermined maximum allowed load, and to a cut-off system (not shown), which cuts off the hydraulic drive to the boom 4, to prevent the boom from being operated when the load exceeds the predetermined maximum load.

When the cage 2 is loaded, the weight carried by the cage initially causes no vertical movement of the cage, since movement is resisted by the pre-stress of the spring 44. The spring is not compressed further until the load is increased to a point where the force exerted on the spring by the load exceeds the predetermined compression force applied to the spring by the bolt 46. In practice therefore the bolt is adjusted to provide a predetermined degree of compression to the spring, which is equivalent to a specified maximum allowed load to be carried by the cage. When the load is less than this predetermined load, no movement of the cage relative to the support frame occurs. However, when the load exceeds the predetermined maximum allowed load, the cage moves downwards relative to the support frame increasing the compression of the spring. Downwards movement of the cage causes rotation of the inner frame element 16 and the actuator arm 34, which actuates the microswitch 48. The actuator arm 34 exaggerates movement of the cage 2, thus increasing the accuracy and sensitivity of the detection system.

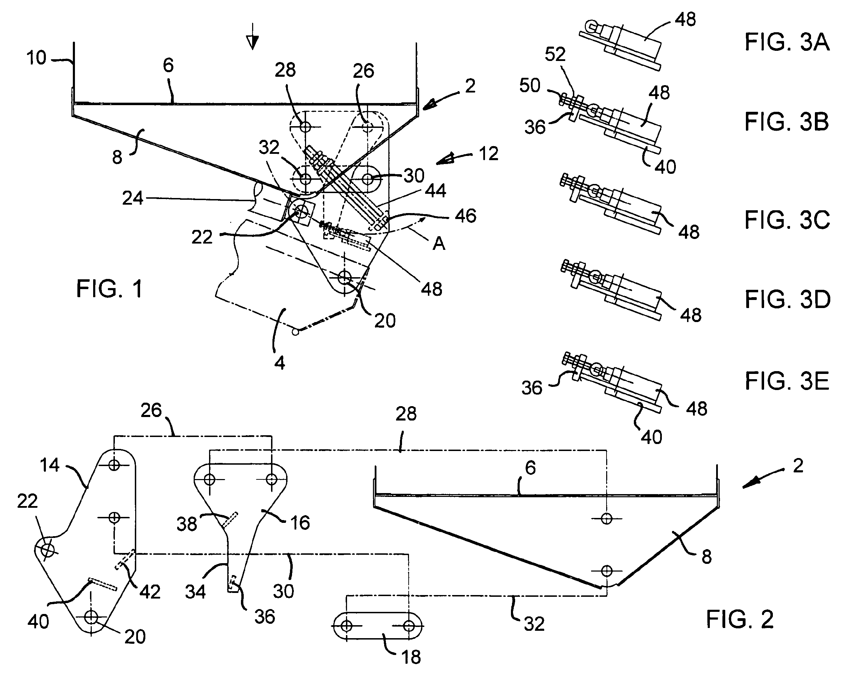

The microswitch 48 has various operating positions as shown in figures 3a-3e. In figure 3a, the microswitch is shown in a free position, for example as when removed from the load detection system. In figure 3b, the microswitch is shown in a pre-loaded condition, in which it is partially compressed by the actuator arm 34, when the cage is unloaded. In this position, the microswitch is in an "off' position, in which the alarm is off and the cage can be operated normally.

Figure 3c shows the microswitch 48 in a third condition, in which the load in the cage just exceeds the pre-stress load of the compressed spring 44, thereby causing a small movement of the actuator arm 34. In this condition, the microswitch has been slightly compressed and thus activates an alarm, warning of an imminent overload situation. However, operation of the hydraulic system is still permitted.

In figure 3d, the microswitch 48 is shown in an overloaded situation, in which the load in the cage exceeds the predetermined allowed maximum. The microswitch has moved to a fourth condition, in which further operation of the hydraulic system is prevented by the alarm and control system.

Finally, in figure 3e the microswitch 48 is shown in a fifth condition where the load in the cage has increased still further. Further vertical movement of the cage 2 relative to the support frame is prevented by contact between the first connecting plate 36 on the actuator arm and the third connecting plate 40 on which the microswitch is mounted. This contact prevents further the load being transmitted to the microswitch, thereby preventing damage to the switch.

The system can be calibrated by placing a load in the cage 2 that corresponds to the intended maximum load. The adjustable screw 50 is then adjusted until it just operates the microswitch 48, and it is then secured in that position with the locknut 52.

## Claims

1. A mobile work platform comprising a cage that is designed to receive one or more human operators, the cage being carried on a boom that is operable to vary the height of the cage, in which the boom includes a link component that is movable by the weight of the cage against the action of resilient biasing means, and detection means operative to generate an output signal in the event that displacement of the link component exceeds a predetermined threshold.

2. A mobile work platform according to claim 1 in which the link component is a pivotable link that is part of a linkage assembly that supports the cage.

3. A mobile work platform according to claim 2 in which the link component is one of a plurality of parallel links that guide movement of the cage.

4. A mobile work platform according to any preceding claim in which the resilient biasing means is pre-stressed to prevent movement of the link component unless the weight of the cage exceeds a predetermined threshold.

5. A mobile work platform according to any preceding claim in which the resilient biasing means comprises a helical compression spring.

6. A mobile work platform according to claim 5 in which the spring is pre-loaded such that it is compressed when the cage is unloaded.

7. A mobile work platform according to claim 6 comprising respective plates abutting end surfaces of the spring, the plates being interconnected by a tie member that passes axially through the spring.

8. A mobile work platform according to claim 7 in which the effective length of the tie member may be adjustable so that the pre-loading of the spring can be varied.

9. A mobile work platform according to claim 8 in which the tie member is threaded.

10. A mobile work platform according to any preceding claim in which the detection means has a number of different operating conditions, the active operating condition being determined by the load within the cage.

11. A mobile work platform according to claim 10 in which the detection means has exactly two operating conditions.

12. A mobile work platform according to claim 10 in which the detection means has a number of operating conditions in excess of two.

13. A mobile work platform according to claim 12 in which an alarm condition is triggered for marginally excessive loading and operation of the cage is prevented for more greatly excessive loading.

14. A mobile work platform according to any preceding claim in which the detection means comprises an electrical device.

15. A mobile work platform according to claim 14 in which the electrical device is a microswitch.

16. A mobile work platform according to claim 14 in which the electrical device is a proximity sensor.

17. A mobile work platform according to any preceding claim in which adjustment means may be provided whereby the relative positions of the detection means and the link component may be altered.

18. A mobile work platform according to claim 17 in which the adjustment means comprises a screwed component of the link component.

19. A mobile work platform according to claim 18 in which the screwed component serves to operate the detection means, the position of the screwed component being adjustable with respect to other parts of the link component.

20. A mobile work platform according to any preceding claim comprising a stop to limit the motion of the link component towards the detection means.

21. A mobile work platform according to any preceding claim, in which the link component includes an actuator arm for actuating the detection means, the link component being constructed and arranged such that movement of the cage produces a larger movement of the actuator arm.
